# EUROPEAN PATENT SPECIFICATION

(11) **EP 3 813 041 B1**
(45) Date of publication and mention of the grant of the patent: **25.02.2026**
(21) Application number: 20199696.4
(22) Date of filing: 21.10.2015
(51) Int. Cl.: G09B 23/28, G09B 7/02, G09B 19/00

(54) **METHODS OF ENHANCING COGNITION AND SYSTEMS FOR PRACTICING THE SAME**
VERFAHREN ZUR VERBESSERUNG DER KOGNITION UND SYSTEM ZUM TRAINIEREN DAFÜR
PROCÉDÉS D'AMÉLIORATION DE LA COGNITION ET SYSTÈMES PERMETTANT DE PRATIQUER CEUX-CI

(30) Priority: 23.10.2014 US 201462067878 P
(43) Date of publication of application: 28.04.2021
(62) Divisional of application: 15852611.1
(73) Proprietor: The Regents of the University of California, Oakland, CA 94607 (US)
(72) Inventor: GAZZALEY, Adam, San Frandisco, CA California 94110 (US)
(74) Representative: Mewburn Ellis LLP

(56) References cited:
- US-A1- 2012 262 484
- US-A1- 2014 135 960

## Description

### INTRODUCTION

Deficits in cognitive control, which encompass the abilities that allow us to accomplish our goals, can lead to significant impairments in cognition, social conduct, and affect regulation, and likely play an important role in a range of mental illnesses including ADHD, PTSD, Major Depressive Disorder, Traumatic Brain Injury, Obsessive Compulsive Disorder, Substance Dependence Disorders, and many others. Targeted cognitive training and physical fitness training are two intervention-based approaches for boosting attention and enhancing deficient cognitive control abilities. Cognitive and physical training approaches have been developed in recent years, with some groups already considering the benefits of trying to couple the two together. See, e.g., Goji Play^{™}. However, these existing approaches do not directly link the two systems together. Rather, cognitive and physical performance in these approaches only indirectly affect the other.

US2014/0135960 discloses a wearable device, display device and system to provide exercise service and methods thereof.

### SUMMARY

Aspects of the present disclosure include a method as set forth in claim 1.

According to certain embodiments, the subject has a cognitive disorder. Cognitive disorders of interest include, but are not limited to, attention deficit hyperactivity disorder (ADHD), traumatic brain injury (TBI), post-traumatic stress disorder (PTSD), obsessive compulsive disorder (OCD), substance dependence disorder (SDD), depression (e.g., Major Depressive Disorder), dementia, Alzheimer's disease, Parkinson's disease, autism, and schizophrenia. In other aspects, the subject is a healthy subject.

In certain aspects, the subject is from an age group selected from preschool, middle childhood, young teen, teenager, younger adult, middle-age adult, and older adult. According to certain embodiments, the methods enhance cognition in the subject (e.g., a healthy subject or a subject having a cognitive disorder).

Also provided are methods of treating a cognitive disorder in a subject. The methods include presenting to a subject an embodied cognitive task that includes a cognitive component and a physical component, assessing the subject's performance on each of the cognitive component and the physical component, and adapting the difficulty level of the embodied cognitive task based on the subject's performance on each of the cognitive component and the physical component, to treat the cognitive disorder. Cognitive disorders that may be treated using the methods of the present disclosure include, but are not limited to, attention deficit hyperactivity disorder (ADHD), traumatic brain injury (TBI), post-traumatic stress disorder (PTSD), obsessive compulsive disorder (OCD), substance dependence disorder (SDD), depression, dementia, Alzheimer's disease, Parkinson's disease, autism, and schizophrenia.

Also provided are methods of diagnosing a subject as having a cognitive disorder. The methods include presenting to a subject an embodied cognitive task that includes a cognitive component and a physical component, assessing the subject's performance on each of the cognitive component and the physical component, and adapting the difficulty level of the embodied cognitive task based on the subject's performance on each of the cognitive component and the physical component. The methods further include comparing the subject's performance on the cognitive component to a threshold cognitive performance value for a cognitive disorder, and diagnosing the subject as having a cognitive disorder when the subject's performance on the cognitive component is less than the threshold cognitive performance value for the cognitive disorder. In certain aspects, the methods are for diagnosing a subject as having attention deficit hyperactivity disorder (ADHD), traumatic brain injury (TBI), post-traumatic stress disorder (PTSD), obsessive compulsive disorder (OCD), substance dependence disorder (SDD), depression, dementia, Alzheimer's disease, Parkinson's disease, autism, or schizophrenia.

According to the methods of the present disclosure, the embodied cognitive task in certain aspects is presented to the subject via a display component, such as a television, a monitor, a high-definition television (HDTV), or a head-up display (HUD). According to certain embodiments, the embodied cognitive task is a video game.

In certain aspects, the cognitive component of the embodied cognitive task may target a cognitive ability of the subject selected from working memory, attention, task-switching, goal management, target search, target discrimination, and any combination thereof. According to certain embodiments, the subject's performance on the cognitive component is assessed by detecting body movement of the subject. The body movement may indicate a reaction time to the cognitive component of the embodied cognitive task. The same or different body movement may indicate a response accuracy to the cognitive component of the embodied cognitive task. In certain aspects, the body movement is detected using a motion capture device (e.g., a motion capture device that includes a depth camera).

According to certain embodiments, The subject's performance on the cognitive component is assessed by detecting neural activity of the subject. Neural activity may be detected using any suitable approach, including but not limited to, electroencephalography (EEG), near-infrared spectroscopy (NIRS), optical imaging, functional magnetic resonance imaging (fMRI), and any combination thereof.

In certain aspects, the physical component of the embodied cognitive task targets a physical ability of the subject selected from the group consisting of: the subject's aerobic energy-generating ability, balance, flexibility, coordination, and any combination thereof. The subject's performance on the physical component may be assessed, e.g., by detecting body movement of the subject. According to certain embodiments, the body movement is detected using a motion capture device, which system may include a depth camera. Alternatively, or additionally, the subject's performance on the cognitive component may be assessed by detecting a physiological measure of the subject. Physiological measures of interest include, but are not limited to, heart rate, heart rate variability, electrodermal response (EDR), pupillary response, and any combination thereof.

The methods of the present disclosure may be computer implemented. As such, aspects of the present disclosure include computer readable media and systems for practicing the methods of the present disclosure. For example, provided are non-transitory computer-readable media that include instructions stored thereon for causing a computer system to implement any of the methods described herein. Systems that include such non-transitory computer-readable media are also provided.

### BRIEF DESCRIPTION OF THE FIGURES

FIG. 1 is a flow diagram illustrating steps of a method according to one embodiment of the present disclosure.
FIG. 2 is a schematic illustration of a system according to one embodiment of the present disclosure.
FIG. 3 is an illustration of the presentation of an embodied cognitive task to a subject according to one embodiment of the present disclosure.
FIG. 4, panels A and B, provide data showing physical outcomes from a training study according to one embodiment of the present disclosure.
FIG. 5, panels A and B, provide data showing cognitive outcomes from the same training study as described for FIG. 4.

### DETAILED DESCRIPTION

Provided are methods that include presenting to a subject an embodied cognitive task that includes a cognitive component and a physical component. The methods further include assessing the subject's performance on each of the cognitive component and the physical component, and adapting the difficulty level of the embodied cognitive task based on the subject's performance on each of the cognitive component and the physical component. In certain aspects, the methods find use in enhancing cognition, physical fitness, or both, in a subject. Also provided are methods of treating and diagnosing cognitive disorders. Systems and computer readable media for practicing the methods of the present disclosure are also provided.

Before the methods of the present disclosure are described in greater detail, it is to be understood that the methods are not limited to particular embodiments described, as such may, of course, vary. It is also to be understood that the terminology used herein is for the purpose of describing particular embodiments only, and is not intended to be limiting, since the scope of the methods will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within by the methods. The upper and lower limits of these smaller ranges may independently be included in the smaller ranges and are also encompassed within by the methods, subject to any specifically excluded limit in the stated range. Where the stated range includes one or both of the limits, ranges excluding either or both of those included limits are also included in the methods.

Unless defined otherwise, all technical and scientific terms used herein have the same meaning as commonly understood by one of ordinary skill in the art to which the methods belong. Although any methods similar or equivalent to those described herein can also be used in the practice or testing of the methods, representative illustrative methods, computer readable media and devices are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the methods, which are, for clarity, described in the context of separate embodiments, may also be provided in combination in a single embodiment. Conversely, various features of the methods, which are, for brevity, described in the context of a single embodiment, may also be provided separately or in any suitable sub-combination. All combinations of the embodiments are specifically embraced by the present disclosure and are disclosed herein just as if each and every combination was individually and explicitly disclosed, to the extent that such combinations embrace operable processes and/or devices. In addition, all subcombinations listed in the embodiments describing such variables are also specifically embraced by the present methods and are disclosed herein just as if each and every such sub-combination was individually and explicitly disclosed herein.

As will be apparent to those of skill in the art upon reading this disclosure, each of the individual embodiments described and illustrated herein has discrete components and features which may be readily separated from or combined with the features of any of the other several embodiments without departing from the scope of the present methods. Any recited method can be carried out in the order of events recited or in any other order that is logically possible.

### METHODS

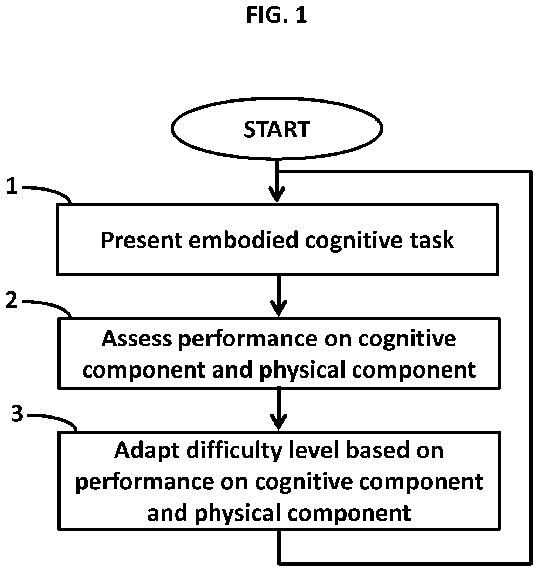

The present disclosure provides methods that include presenting to a subject an embodied cognitive task that includes a cognitive component and a physical component. The methods further include assessing the subject's performance on each of the cognitive component and the physical component, and adapting the difficulty level of the embodied cognitive task based on the subject's performance on each of the cognitive component and the physical component. These steps are shown as steps 1, 2 and 3, respectively, in FIG. 1. In certain aspects, the methods find use in enhancing cognition, physical fitness, or both, in a subject. Also provided are methods of treating and diagnosing cognitive disorders.

Aspects of the methods of the present disclosure include presenting an embodied cognitive task to a subject. According to certain aspects, the subject is a human subject, e.g., a female or male human subject. Human subjects of interest include children and adults. In certain aspects, the human subject is from 4 years old to 100 years old, such as from 8 years old to 100 years old, from 9 years old up to 90 years old, from 10 years old up to 80 years old, from 11 years old up to 75 years old, or from 12 years old up to 70 years old. According to certain embodiments, the human subject is a child (newborn up to 18 years old). Children of interest include infants (newborn up to 1 year old), toddlers (1 year old up to 3 years old), preschoolers (3 years old up to 4 years old), children in middle childhood (4 years old up to 11 years old, such as 6 years old up to 8 years old, or 8 years old up to 11 years old), young teens (11 years old up to 14 years old) and teenagers (14 years old up to 18 years old). When the subject is a human adult, the subject may be a younger adult (an 18-30 year-old adult, e.g., a 21-28 year-old adult)), a middle-age adult (a 31-49 year-old adult), or an older adult (a 50 year-old or older adult (e.g., a 57-75 year-old adult)).

The subject may have a cognitive disorder, or the subject may be a healthy subject. As used herein, a "cognitive disorder" is a disorder that affects one or more mental processes, including impairments in one or more aspects of cognitive control, such as memory (e.g., working memory), attention, task-switching, goal management, target search, target discrimination, self-regulation, language comprehension and emotional processing. Such disorders may be accompanied by personality and behavioral changes. A "healthy subject" is a subject who does not have a cognitive disorder.

By "task" is meant a goal and/or objective to be accomplished by the subject who provides a response to a particular stimulus. As used herein, an "embodied cognitive task" is a task the completion of which requires both a cognitive and a physical response from a subject. In certain aspects, the physical response to the cognitive task is movement of the subject, e.g., movement of the subject's arms and/or legs.

According to certain embodiments, the embodied cognitive task is presented to the subject in the form of a video game. Embodied cognitive tasks presented in the form of a video game may be presented to the subject via a display component, e.g., a television, a monitor, a high-definition television (HDTV), a projection screen, a head-up display (HUD), or any other suitable display for presentation of the embodied cognitive task in the form of a video game.

The video game may include engaging game elements (e.g., an immersive 3-dimensional environment, music, art work, adaptivity, feedback, rewards/incentives, and/or the like). The video game context may encourage the subject to engage more attentional resources to the embodied cognitive task, which may facilitate cognitive enhancement in the subject. When provided as part of a cognitive training program, the video game context can provide incentives for a subject to pay attention and/or complete the training. That is, the interest and goal orientation created by the game context provide incentives to continue training for longer periods of time than would generally be supported by the less engaging training task on its own. Game specific features that can increase incentive and interest of the subject may include, but are not limited to, bonus points, in-game reward or penalty, such as a graphical or auditory representation thereof, rewards or penalties that scale with difficulty level or time spent, real life rewards, etc. Additional game elements to enhance engagement are known to those skilled in the art of video gaming, board games, cognitive paradigms, fitness/sports instruction, and educational programs.

The embodied cognitive task may be designed such that the cognitive component of the task targets (or interrogates) one or more cognitive abilities of the subject. For example, the cognitive component may target a cognitive ability of the subject selected from working memory, attention, task-switching, goal management, target search, target discrimination, and any combination thereof.

As summarized above, the methods include assessing the subject's performance on the cognitive component. Because the embodied cognitive task is a cognitive task that requires a physical response from the subject, in certain aspects the subject's performance on the cognitive component is assessed based on the subject's physical response to the task. In certain aspects, assessing the subject's performance on the cognitive component includes detecting body movement of the subject. The body movement may indicate the subject's reaction time to the task, accuracy on the task, or both, enabling an assessment of the subject's performance on the cognitive component, the physical component, or both.

According to certain embodiments, the subject's body movement is detected using sensors (e.g., wired or wireless sensors) worn on the body of the subject. Such sensors may be worn on one or both of the subject's arms, one or both of the subject's legs, and/or any other useful location on the subject's body for detecting the subject's movement and/or position while performing the embodied cognitive task. In certain aspects, the sensors are "wireless" sensors that communicate wirelessly with the system being implemented to the carry out the method. Such wireless sensors are known and include the PrioVR motion-tracking body suits available from YEI Corporation (Portsmouth, Ohio).

In certain aspects, a motion/position capture device is employed to detect the subject's body movement while responding to the embodied cognitive task. A non-limiting example of a motion capture device includes a video camera, such as an RGB video camera and/or a depth camera. Movements made by the subject are captured in a sequence of images and then processed. The motion capture device, alone or in conjunction with a second component (e.g., a video game console) may perform functions including, but not limited to, detecting and performing gesture recognition on a user's movements and monitoring direction and relative distance moved by a user. Images of the user's movements may be stored for analysis.

According to certain embodiments, the motion/position capture device includes a depth camera that can sense distance between an imaging sensor in the camera and objects in the camera's field of view, in order to acquire a depth image of the subject. Depth images, color images, or both may be captured. If both color and depth images are captured, the color and depth images may be acquired simultaneously by a camera with two lenses, one for acquiring color images and one for acquiring depth images. A color image is a digital representation of an image which contains multiple channels, each channel corresponding to a different color. In certain aspects, three channels are used, and each channel corresponds to one of the colors red, green, and blue. However, any other suitable number of colors and color selection may be assigned to the multiple channels. Each channel is composed of an identical number of pixels, and each pixel has an intensity value between zero and a maximum number. The maximum number may vary depending upon the application of the images. The value of each pixel corresponds to the contribution of that color channel at each pixel's location.

A depth image may contain a single channel composed of the same number of pixels as each color channel. The value of each pixel in a depth image corresponds to the distance between the camera lens and the user at each corresponding pixel's location. Different approaches may be employed for generating depth images, including time of flight, stereoscopic vision, and triangulation. The color images and the depth images may be analyzed and processed independently.

The subject's body movement while responding to the embodied cognitive task occurs over a span of time. Because it is not known when the subject will begin to make any given body movement, images of the subject may be taken sequentially, and multiple images over a span of time may be recorded for analysis. The multiple images may each include a color and depth image as discussed above. The following steps apply to each of the multiple images in the sequence.

The acquired color and depth images may be used to locate feature points of interest on the subject's body. Feature points of interest may include joints and locations corresponding to, for example, the user's left foot, left knee, left hip, left hand, left elbow, left shoulder, head, right shoulder, right elbow, right hand, right hip, right knee, right foot, and/or any other useful feature points for detecting movement of the subject.

Three-dimensional coordinates for each one of the feature points of interest may be computed from color and/or depth images. The coordinate locations for each of the feature points of interest may be stored for the frame corresponding to co-acquired color and depth images.

Classification and/or evaluation of the subject's recorded movements may be accomplished by comparing the movements with movement data stored in a movement library. Each movement in the library may consist of a sequence of images covering the period of time required to perform the movement, with a uniform time lapse occurring between images in the sequence with a minimum number of sequential images sufficient to capture the movement. Further details regarding motion capture devices for detecting body movement are found, e.g., in U.S. Patent No. 8,113,991, U.S. Patent Application Publication No. US 2010/0199228, U.S. Patent Application Publication No. US 2013/0342527, and U.S. Patent Application Publication No. US 2014/0244008.

Motion/position capture devices that find use in detecting body movement are available and include Microsoft's Kinect motion capture device (e.g., the Microsoft Kinect for Windows v2), Sony's EyeToy^{®} motion capture device, and the like.

According to certain embodiments, the subject's performance on the cognitive component is assessed by detecting neural activity of the subject. Suitable approaches for detecting neural activity (e.g., neural activity relating to cognitive control functions, or any other neural activity of interest) include, but are not limited to, electroencephalography (EEG), near-infrared spectroscopy (NIRS), optical imaging, functional magnetic resonance imaging (fMRI), and any combination thereof. For example, in certain aspects, neural activity of the subject is recorded using a mobile EEG device (e.g., an EEG cap, such as a 64-channel EEG cap (or "headset")) worn by the subject to enable recording of voltage fluctuations resulting from ionic current flows within the neurons of the brain at desired intervals, including in real-time, during presentation of the embodied cognitive task and the subject's response thereto. For example, neural activity data can be collected from motor and sensory areas of the brain to see how the core perceptual and action processes are functioning, and use this information to drive the adapting step of the methods (e.g., to drive an adaptive algorithm that effects the adaptation of the difficulty level of the task). Alternatively, or additionally, neural signals from cognitive control areas of the brain in the prefrontal cortex and parietal cortex can be used to assess control processes, such as attention, working memory, goal management, and decision making, which can also be used to drive the adapting step (e.g., drive the adaptive algorithms) of the methods.

As summarized above, the methods include assessing the subject's performance on the physical component. The embodied cognitive task may be designed such that the physical component targets one or more physical abilities of interest. According to certain embodiments, the physical component targets a physical ability of the subject selected from the subject's aerobic energy-generating ability (e.g., cardiorespiratory fitness), balance, flexibility, coordination, and any combination thereof.

In certain aspects, the subject's performance on the physical component is assessed by detecting body movement of the subject. Any convenient sensors or devices may be employed to detect the subject's body movement, including wired or wireless motion sensors worn by the subject as described above, a motion/position capture device as described above, e.g., a motion/position capture device that includes a depth camera to capture depth images of feature points of interest on the subject's body, and the like. Detecting movement and/or position of the subject's body finds use in assessing the subject's performance with respect to balance, flexibility, coordination, and the like. The subject's movements/positions may be compared to reference movement/position data to assess the subject's performance on the physical component of the embodied cognitive task.

Also encompassed by embodiments of the methods is the use of pressure-sensing devices for assessing the subject's performance on one or more aspects of the cognitive and/or physical components. For example, the methods may include presenting the embodied cognitive task to a subject while the subject is on a pressure-sensitive mat that detects, e.g., the distance and/or timing of the subject's steps to assess balance, flexibility, coordination, and/or the like. Also by way of example, if the embodied cognitive task requires the subject to throw an object (e.g., a ball) at a real or virtual target, a wall in front of the subject may include pressure sensors to detect the timing and/or position of the object's contact with the wall to assess, e.g., the subject's reaction time, target discrimination ability, coordination, such as hand-eye coordination, and/or the like. In certain aspects, the subject's balance is assessed by requiring the subject to perform the embodied cognitive on a balance board.

Alternatively or in addition to any of the physical assessment approaches described above, the subject's performance on the physical component of the embodied cognitive task may be assessed by detecting one or more physiological measures of the subject. Non-limiting examples of physiological measures which may be detected to assess the subject's performance include heart rate, heart rate variability, VO₂ Max, electrodermal response (EDR), pupillary response, and any combination thereof. In certain aspects, heart rate and/or heart rate variability are detected using a mobile heart rate detection device, such as a device worn by the subject during presentation of the embodied cognitive task and the subject's response thereto. According to certain embodiments, the device is worn on the subject's wrist, an example of which includes the Peak fitness tracker by Basis, Inc. and the V800 sports watch by Polar Electro, Inc.

Aerobic energy-generating ability/cardiorespiratory endurance may be assessed by determining the subject's VO₂ Max (which may be expressed as liters of oxygen per minute (L/min) or milliliters of oxygen per kilogram of body mass per minute (mL/(kg·min), or the like). VO₂ Max may be determined by measuring ventilation and oxygen and carbon dioxide concentration of the inhaled and exhaled air. VO₂ Max is reached when oxygen consumption remains at steady state despite an increase in workload. VO₂ Max may also be estimated using the Uth-Sørensen-Overgaard-Pedersen estimation, the Cooper Test, or the like.

Electrodermal responses of interest for assessing the subject's performance on the physical component include, but are not limited to, the galvanic skin response (GSR). In certain aspects, GSR is detected using a mobile GSR detection device, such as a device worn by the subject during presentation of the embodied cognitive task and the subject's response thereto. According to certain embodiments, the device is worn on the subject's wrist, an example of which includes the Peak fitness tracker by Basis, Inc.

Suitable approaches for detecting the subject's pupillary response include capturing images of one or both of the subject's pupils during presentation of the embodied cognitive task and the subject's response thereto. In certain aspects, a motion capture device employed to detect the subject's body movement is also used to detect the users pupillary response while responding to the embodied cognitive task. Pupillary responses of interest include constriction (miosis) and/or dilation (mydriasis).

As summarized above, the methods include adapting the difficulty level of the embodied cognitive task. In certain aspects, the difficulty level is adapted based on the subject's performance on each of the cognitive component and the physical component. Algorithms may be employed to adapt the difficulty level based on the subject's performance on the cognitive and physical components. Adaptive methodologies/algorithms that may be employed include, but are not limited to, parameter estimation by sequential testing (PEST), maximum-likelihood procedures, and staircase procedures.

According to certain embodiments, adapting the difficulty level of the embodied cognitive task is achieved using PEST, which is characterized by an algorithm for threshold searching that changes both step sizes and direction (i.e., increasing and decreasing level) across a set of trials. Changes in step size are used to focus the adaptive track ever more finely, stopping the track when the estimate has been adequately defined. The final estimate is simply the final value determined by the trial placement procedure. The PEST algorithm is designed to place trials at the most efficient locations along the stimulus axis in order to increase measurement precision while minimizing the number of trials required to estimate a threshold.

In certain aspects, adapting the difficulty level of the embodied cognitive task is effected using maximum-likelihood procedures. In maximum-likelihood procedures, sets of stimulus-response trials are fit with an ogival function and subsequent trial placement and threshold estimation is taken from those fitted functions. Maximum-likelihood procedures are characterized by stimulus placement on each trial, driven by consulting the current best estimate of the entire underlying psychometric function after every stimulus-response trial. As the adaptive track grows in length, the estimated function becomes better defined by the collection of data points generated from previous trials. After each trial, the set of stimulus levels and the proportion of correct responses associated with each level are combined to form a psychometric function. The individual points are fitted with an ogival function and a current estimated threshold level is extracted. A new psychometric function is generated after each trial or set of trials, and subsequent trials are placed at a targeted performance level on the most up-to-date function. A maximum-likelihood fitting algorithm is typically used with this type of procedure.

According to certain embodiments, adapting the difficulty level of the embodied cognitive task is achieved using an adaptive staircase algorithm/procedure. Staircase procedures generally use the previous one or more responses within an adaptive track to select the next trial placement, then provide a threshold estimate in a variety of ways, e.g., by averaging the levels at the direction reversals in the adaptive track (i.e., the turnaround points). Up-down staircases call for a reduction in stimulus level when the subject's response is positive and an increase in stimulus level when the response is negative. Beginning at a level above threshold, positive responses lead to continued decreases in stimulus level until a negative response occurs. This triggers a reversal in the direction of the track, and levels on subsequent trials increase until the next change in response. The up-down staircase procedure targets the 50% performance level on a psychometric function that extends from 0% correct performance at chance to 100% correct performance. That is, the track targets the stimulus level for which the probability of a correct response equals the probability of an incorrect response or, equivalently, the level at which the track would move up or down on the stimulus axis with equal probability. The value of this type of procedure is in the very few assumptions necessary for its implementation. To target a higher performance level, the sequence for a downward movement may be two or more positive responses, and the sequence for an upward movement may remain at one negative response. This example is a two-down, one-up procedure, which targets the 70.7% level on the psychometric function. Recalling that the probability of the down sequence must equal the probability of an up sequence, we see that a positive response to two consecutive trials must occur in order to move the track downward. If p is the probability of a positive response on a given trial, then p x p must equal .50, and therefore the target probability is .707. Similarly, a three-down, one-up transformation leads to a performance target of .794 (p³ = .50; the cube root of .50 is .794).

When the embodied cognitive task is presented to the subject as part of a cognitive training program, such adaptive algorithms may augment the training experience to keep interest and ability levels properly titrated for each individual, and also make the training program adaptable to any target population, ranging from adolescents with ADHD to more sedentary older adults. In certain aspects, the demands in each domain (the cognitive component and the physical component) are "tethered," such that the adaptation prevents tradeoffs. For example, according to certain embodiments: improvement in the subject's physical fitness results in an adaptation of the embodied cognitive task such that the cognitive component is more difficult as compared to the previously presented embodied cognitive task; and/or improvement in the subject's cognitive abilities (e.g., one or more aspects of the subject's cognitive control abilities) results in an adaptation of the embodied cognitive task such that the physical component is more difficult as compared to the previously presented embodied cognitive task.

According to certain embodiments, the methods include providing feedback (e.g., positive (e.g., rewards) and/or negative feedback) to the subject based on the subject's performance on the embodied cognitive task. For example, when the embodied cognitive task is presented as part of a video game, feedback in the form of an in-game reward (e.g., bonus points) or penalty, such as a graphical or auditory representation thereof, may be provided to the subject upon the subject performing at a specified level, e.g., surpassing a particular score/number of points, "passing a level", and the like. According to certain embodiments, the feedback is "tethered" to the subject's performance across the cognitive and physical components. For example, in certain aspects, the methods of the present disclosure include rewarding a subject only when a threshold level of performance on each of the cognitive and physical components is achieved. According to such aspects, regardless of how well the subject performs on one of the components, a reward will not be presented to the subject if performance on the other component is less than a threshold level for that component. Positive and/or negative feedback may be provided on multiple time scales with respect to game play for both cognitive and physical components in addition to the adaptivity mechanics so that the game will challenge the subjects right at the "sweet spot" of their abilities, never becoming too easy, so as to be boring, or too difficult, as to be frustrating.

In certain aspects, the embodied cognitive task may be presented to the subject as part of a cognitive training program. According to certain embodiments, the training program includes presenting 2 or more, 3 or more, 4 or more, 5 or more, 10 or more, 15 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, or 100 or more embodied cognitive tasks of a selected duration, the difficulty levels of which may, in certain aspects, be adapted based on the subject's performance on each of the cognitive and physical components during presentation of a preceding embodied cognitive task at a selected difficulty level. Such a training program may include presenting one or more embodied cognitive tasks to a subject each day over a number of days (e.g., a number of consecutive or nonconsecutive days), such that the training program includes multiple "sessions" where the one or more presentations of an embodied cognitive task during a day constitutes a single session. The training program may be presented for any number of days, e.g., until a desired level of cognitive enhancement is achieved. In certain aspects, the entire training program is presented on a single day. In other aspects, the entire training program lasts from 2 to 7 days, from 8 to 14 days, from 15 to 21 days, from 22 to 28 days, or any other number of days suitable for achieving a desired result.

The methods of the present disclosure may include a step prior to the first presenting step. For example, where the method is carried out for the first time for a subject, the method may encompass a thresholding step, an assessment, instruction, and/or demonstration.

A thresholding step includes presenting to a subject an embodied cognitive task in one or more trials. This thresholding step helps determine an initial performance level of the subject on the embodied cognitive task. Another purpose of the thresholding is to determine a difficulty level to carry out an assessment. Thresholding to a specific difficulty level can be useful in tailoring the methods to an individual because each subject can have variable baseline abilities to perform the embodied cognitive task (e.g., older adults perform certain tasks at a lower performance level when the task is matched in difficulty).

A difficulty level to carry out an assessment may be a level at which the subject performs the task with a pre-determined percentage of accuracy (e.g., 80%), optionally within a pre-selected maximum response time. The initial difficulty level may be a default difficulty level for a category of subjects (e.g. average for an age range), a lowest level of difficulty, or a level comparable based on the subject's prior assessment. The difficulty levels then can change until the subject performs with a specific level of accuracy. In an alternative method, a single training session run can use adaptive thresholding methods (such as psychometric staircase algorithms) to quickly threshold the individual at a specific performance level.

The threshold level can be personalized to the subject. For example, in certain aspects, a threshold level for the first time is a level at which the subject can perform the embodied cognitive task with an accuracy of about 50% or more, about 55% or more, about 60% or more, about 65% or more, about 70% or more, about 75% or more, about 80% or more, about 85% or more, or about 90% or more. After a thresholding step, the methods may include an assessment before, during and/or after a training session or training program.

When practicing the methods of the present disclosure, any thresholding step and/or assessment may be with respect to: the subject's overall performance on the cognitive task, the subject's performance on the cognitive component alone, the subject's performance on the physical component alone, the subject's performance on the cognitive component and the physical component separately, or any combination thereof.

In certain aspects, a pre- and/or post-training cognitive assessment is carried out. Cognitive assessment may include assessing: attention (e.g., using Test of Variables of Attention (T.O.V.A.), and/or Continuous Performance Task); attentional capacity (e.g., using Multiple Object Tracking); working memory (e.g., using a Change Detection Task, Filter Task, 2- & 3-back task, and/or Delayed Recognition Working Memory Task); processing speed (e.g., using a WAIS-III Digit Symbol Task, UFOV with eccentricity, and/or a Reaction Time Task); or any combination thereof. Lifestyle, personality and/or mood questionnaires, including those used in the ACTIVE study, may be used to establish rates of media multitasking, etc. Each of these measures may be supplemented with neural recordings using EEG or fMRI to better understand the underlying neural mechanisms associated with training-based changes in brain plasticity. Cognitive outcomes resulting from the methods of the present disclosure may be assessed by comparing the results of pre- and/or post-training cognitive assessments.

In certain aspects, a pre- and/or post-training physical assessment is carried out. The physical assessment may be carried out in addition to any cognitive assessment. Physical assessment may include assessing: height and weight; heart rate variability; blood pressure; body fat by skinfold measurements; vertical jump; agility (e.g., using the Hexagon Agility Test); and maximal aerobic capacity (e.g., as determined by VO₂ max); or any combination thereof. Physical outcomes resulting from the methods of the present disclosure may be assessed by comparing the results of pre- and/or post-training physical assessments. Each of these measures may be supplemented with blood or saliva measures assessing physiological characteristics facilitating a better understand of the underlying physical mechanisms associated with training-based changes.

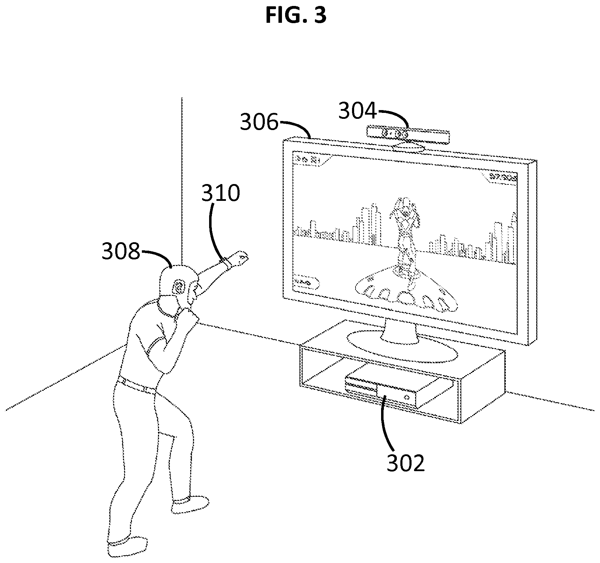

FIG. 3 provides an illustration of the presentation of an embodied cognitive task to a subject according to one embodiment of the present disclosure. In this example, the embodied cognitive task is presented to the subject in the form of a video game. The system for presenting the embodied cognitive task to the subject includes video game console 302, motion capture system 304 and display component 306. The subject responds to the embodied cognitive task using arm and/or leg movements which permit an assessment of the subject's performance on the cognitive and/or physical components of the embodied cognitive task. In this example, the subject is wearing EEG headset 308 for detection of neural activity while responding to the task. In addition, worn on the subject's wrist is wearable device 310 that detects the heart rate and/or galvanic skin response (GSR) of the subject while responding to the task.

As summarized above, according to certain embodiments, the methods of the present disclosure enhance cognition in the subject. By "enhance cognition" is meant at least one aspect of the subject's cognition is improved as a result of the presenting, assessing, and adapting steps (or two or more iterations of such steps, e.g., as part of a training session or training program). Aspects of the subject's cognition that may be enhanced include, but are not limited to, the subject's memory (e.g., working memory), attention, task-switching ability, goal management ability, target search ability, target discrimination ability, and/or the like.

According to certain embodiments, enhancement of the subject's cognition is determined by performing a pre-training assessment and a post-training assessment. The methods may also include one or more of the assessments intermittently throughout the training (e.g., inter-trial or inter-session). An assessment may include presenting an embodied cognitive task and evaluating the performance of the subject. The assessment may be different from a training session in that it does not seek to train the subject. In one embodiment, unlike a training session, the difficulty level from trial to trial in an assessment does not change or adapt to the performance of the subject. Rather, the difficulty level for assessment purposes remains the same (e.g. at the difficulty level determined by a thresholding step). In another embodiment, the assessment does include an adaptation in difficulty level, e.g., by methods known in the art of psychometric analysis (e.g., staircase procedures and/or maximum likelihood procedures) to adaptively determine the ability of the subject. In either case, the primary purpose of the assessment is to evaluate the performance of the individual as opposed to train that performance.

The assessment described above can be conducted before and/or after a training session or training program. The steps involved in a post-training assessment are the same as those of a pre-training assessment described above, except that in a pre-training assessment, the data are used to determine the ability and/or performance of a subject prior to training. In a post-training session, however, the data analyzed may include data collected not just in the assessments but also during the training program. Additionally, the post-training assessment may be used as feedback to the subject, as well as feedback to the training program, as a control by which to direct the advancement of the next training session.

The analysis also reflects the performance and ability of the subject after training. In other words, post-training assessment can compare the performance of the subject post-training to that prior to training and assess the impact of training on the cognitive ability of the subject, the physical ability of the subject, or both. When one or more cognitive abilities of the subject is improved post-training (as revealed by comparing the cognitive ability during a post-training cognitive ability assessment to the cognitive ability during a pre-training cognitive ability assessment), the subject's cognition has been enhanced by the method. Similarly, when one or more physical abilities of the subject is improved post-training (as revealed by comparing one or more physical abilities during a post-training physical ability assessment to the physical ability during a pre-training physical ability assessment), the subject's physical ability has been enhanced by the method. Cognitive abilities that may be assessed include but are not limited to working memory, attention, task-switching, goal management, target search, target discrimination, and/or the like. Physical abilities that may be assessed include, but are not limited to, VO₂ Max, balance, control of movement, coordination, flexibility, cardiovascular health, stress levels (as measured by, e.g., cortisol levels and/or skin conductivity), sleep measurements, real-world performance, productivity and/or wellness.

### Therapeutic Methods

As summarized above, provided by the present disclosure are methods of treating a cognitive disorder in a subject. The treatment methods include presenting to a subject having a cognitive disorder an embodied cognitive task comprising a cognitive component and a physical component, and assessing the subject's performance on each of the cognitive component and the physical component. The methods further include adapting the difficulty level of the embodied cognitive task based on the subject's performance on each of the cognitive component and the physical component, to treat the cognitive disorder in the subject. The presenting, assessing, and adapting steps may be performed using any of the approaches described hereinabove.

With the goal of enhancing cognition in the subject, it can be desirable to experimentally determine the efficacy of a training session or program. Suitable methods of experimental testing include types of studies known to test the efficacy of cognitive, behavioral, or pharmacological intervention, including pilot human studies and clinical trials. One efficacy-testing method suitable for the methods of the present disclosure is the administration of pre-training and post-training assessments which allow for the determination of whether training has led to a measurable change in one or more cognitive abilities of the subject (e.g., one or more cognitive control abilities).

According to certain embodiments, the pre-training and post-training assessment is comprised of general cognitive functions, which pertain to both healthy individuals and individuals that have experienced or are at risk of experiencing cognitive deficits, including clinical patient populations. Such suitable tests include those that test any specific functions of a range of cognitions in cognitive or behavioral studies, including tests for perceptive abilities, reaction and other motor functions, visual acuity, long-term memory, working memory, short-term memory, logic, decision-making, and the like. Specific examples of such measurements include but are not limited to TOVA, MOT (motion-object tracking), SART, CDT (Change detection task), UFOV (useful Field of view), Filter task, WAIS digit symbol, Troop, Simon task, Attentional Blink, N-back task, PRP task, task-switching test, Flanker task, and any combinations thereof.

Alternatively, or additionally, the pre-training and post-training assessment include tests that measure improvement on actual functional activities of daily living. Examples can include tests that are specifically constructed or validated to measure such outcomes, such as Activities of Daily Living that are used in clinical trials of elderly populations, or similar simple measurements such as the ability to perform a directed task, reading or conversational comprehension, efficiency in a workplace environment, and the like.

Alternatively, or additionally, the pre-training and post-training assessment includes tests that measure improvement on symptoms or functions relevant to a specific disease or condition. Suitable types of tests include those that objectively measure symptom severity or biomarkers of a disease or condition, tests that use subjective clinician or observer measurement of symptom severity, and tests that measure cognitive functions known to be correlated with disease states. Examples of such tests include but are not limited to assessment scales or surveys such as the Mini Mental State Exam, CANTAB cognitive battery, Repeatable Battery for the Assessment of Neuropsychological Status, Clinical Global Impression scales relevant to specific conditions, Clinician's interview-Based Impression of Change, Severe Impairment Battery, Alzheimer's Disease Assessment Scale, Positive and Negative Syndrome Scale, Schizophrenia Cognition Rating Scale, Conners Adult ADHD Rating Scales, Hamilton Rating Scale for Depression, Hamilton Anxiety Scale, Montgomery-Asberg Depressing Rating scale, Young Mania Rating Scale, Children's Depression Rating Scale, Penn State Worry Questionnaire, Hospital Anxiety and Depression Scale, Aberrant Behavior Checklist, and Activities of Daily Living scales; physiological tests that measure internal markers of disease or health such as detection of amyloid beta, cortisol and other stress response markers; and brain imaging studies (for example fMRI, PET, etc.) that assess a condition based on presence of specific neural signatures.

Alternatively, or additionally, the pre-training and post-training assessment includes survey or questionnaire-style tests that measure a subject's self-reported perception of themselves. These can include self-report scales of healthy function or feelings, or disease function or symptoms. Examples of suitable self-report tests include but are not limited to ADHD self-report scale, Positive and Negative Affect Schedule, Depression Anxiety Stress Scales, Quick Inventory of Depressive Symptomatology, PTSD Checklist, and any other types of surveys that can be conducted for a subject to report on their general feelings of symptoms of a condition or satisfaction with real-world functional status or improvement.

### Diagnostic Methods

The present disclosure provides diagnostic methods. The methods may be used to diagnose a subject as having a condition (e.g., a cognitive disorder), diagnosing the onset of a condition in a subject, diagnosing a change in a condition of the subject, diagnosing a subject as no longer having a condition, or any combinations thereof.

As summarized above, in certain aspects, the present disclosure provides methods of diagnosing a subject as having a cognitive disorder. The diagnostic methods include presenting to a subject an embodied cognitive task that includes a cognitive component and a physical component, assessing the subject's performance on each of the cognitive component and the physical component, and adapting the difficulty level of the embodied cognitive task based on the subject's performance on each of the cognitive component and the physical component. The presenting, assessing, and adapting steps may be performed using any of the approaches described hereinabove.

The diagnostic methods further include comparing the subject's performance on the cognitive component to a threshold cognitive performance value for a cognitive disorder, and diagnosing the subject as having a cognitive disorder when the subject's performance on the cognitive component is less than the threshold cognitive performance value for the cognitive disorder. In certain aspects, the subject's performance on the cognitive component may be determined during a single training session or multiple training sessions using adaptive thresholding methods (such as psychometric staircase algorithms) to threshold the individual at a specific performance level, which performance level may then be compared to a threshold cognitive performance level for the cognitive disorder in order to determine whether the individual has the cognitive disorder.

Aspects of the diagnostic methods include diagnosing a subject as having a cognitive disorder selected from, but not limited to, attention deficit hyperactivity disorder (ADHD), traumatic brain injury (TBI), post-traumatic stress disorder (PTSD), obsessive compulsive disorder (OCD), substance dependence disorder (SDD), depression, dementia, Alzheimer's disease, Parkinson's disease, autism, and schizophrenia.

### COMPUTER READABLE MEDIA AND DEVICES/SYSTEMS

Aspects of the present disclosure further include computer readable media and devices/systems. In certain aspects, provided are non-transitory computer readable media including instructions stored thereon for causing a computer device/system to implement the methods of the present disclosure, including any embodiments of the methods described elsewhere herein. For example, the computer readable medium may include instructions to cause the computer device/system to present to a subject an embodied cognitive task including a cognitive component and a physical component, assess the subject's performance on each of the cognitive component and the physical component, and adapt the difficulty level of the embodied cognitive task based on the subject's performance on each of the cognitive component and the physical component.

Non-transitory physical computer readable media of the present disclosure include, but are not limited to, disks (e.g., magnetic or optical disks), solid-state storage drives, cards, tapes, drums, punched cards, barcodes, and magnetic ink characters and other physical medium that may be used for storing representations, instructions, and/or the like.

In certain aspects, the non-transitory physical computer readable media is suitable for use with a video game console, such as Microsoft's Xbox One video game console operably connected to a Kinect motion capture device capable of monitoring the subject's movement during performance of the embodied cognitive task. According to certain embodiments, the instructions for causing a computer device/system to implement the methods of the present disclosure are downloaded to the computer device/system (e.g., a video game console) from a remote server, such that the non-transitory physical computer readable medium is part of the computer device/system, e.g., the non-transitory physical computer readable medium is the hard drive of the computer device/system. In certain aspects, the instructions for carrying out the methods of the present disclosure are present on a remote server.

Also provided are computer devices/systems that carry out the methods of the present disclosure. In certain aspects, the computer device/system includes a non-transitory computer readable medium according to any of the embodiments described above. According to certain embodiments, the computer device does not include instructions for carrying out the methods of the present disclosure, but rather serves as a portal for presenting a cognitive training program the instructions for which are stored on a remote server.

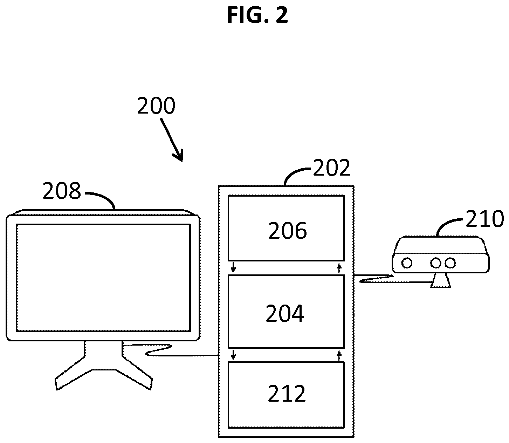

FIG. 2 schematically illustrates an embodiment of a system (as shown, system 200) that finds use in practicing methods according to certain aspects of the present disclosure. Computing subsystem 202 is shown and may take the form of a video game console, mainframe computer, server computer, desktop computer, laptop computer, tablet computer, home-entertainment computer, network computing device, mobile computing device, mobile communication device (e.g., smart phone), etc. Computing subsystem 202 includes a logic subsystem 204 and a storage subsystem 206. The system also includes a display component 208, an input-device (e.g., a motion capture device 210), communication subsystem 212, and any other useful components.

Logic subsystem 204 may include one or more processors configured to execute software instructions. Additionally or alternatively, the logic subsystem may include one or more hardware or firmware logic machines configured to execute hardware or firmware instructions. The processors of the logic subsystem may be single-core or multi-core, and the programs executed thereon may be configured for sequential, parallel or distributed processing. The logic subsystem may include individual components that are distributed among two or more devices, which can be remotely located and/or configured for coordinated processing. Aspects of the logic subsystem may be virtualized and executed by remotely accessible networked computing devices configured in a cloud-computing configuration.

Storage subsystem 206 includes one or more physical, non-transitory, devices configured to hold data and/or instructions executable by the logic subsystem to implement the methods of the present disclosure. When such methods and processes are implemented, the state of storage subsystem 206 may be transformed, e.g., to hold different data.

Storage subsystem 206 may include removable media and/or built-in devices. Storage subsystem 206 may include optical memory devices (e.g., CD, DVD, HD-DVD, Blu-Ray Disc, etc.), semiconductor memory devices (e.g., RAM, EPROM, EEPROM, etc.) and/or magnetic memory devices (e.g., hard-disk drive, floppy-disk drive, tape drive, MRAM, etc.), among others. Storage subsystem 206 may include volatile, nonvolatile, dynamic, static, read/write, read-only, random-access, sequential-access, location-addressable, file-addressable, and/or content-addressable devices.

Storage subsystem 206 may include one or more physical, non-transitory devices. However, in some embodiments, aspects of the instructions described herein may be propagated in a transitory fashion by a pure signal, e.g., an electromagnetic or optical signal, etc. that is not held by a physical device for a finite duration. Furthermore, data and/or other forms of information pertaining to the present disclosure may be propagated by a pure signal.

According to certain embodiments, aspects of logic subsystem and of the storage subsystem may be integrated together into one or more hardware-logic components. Such hardware-logic components may include field-programmable gate arrays (FPGAs), program- and application-specific integrated circuits (PASIC/ASICs), program- and application-specific standard products (PSSP/ASSPs), system-on-a-chip (SOC) systems, and complex programmable logic devices (CPLDs), for example.

Display component 208 may be used to present an embodied cognitive task present in instructions held by storage subsystem 206. The embodied cognitive task may take the form of a video game, a graphical user interface (GUI), and the like. As the methods change the data held by the storage subsystem, and thus transform the state of the storage subsystem, the state of the display component may likewise be transformed to visually represent changes in the underlying data. The display component may include one or more display devices utilizing virtually any type of technology. Such display devices may be combined with the logic subsystem and/or the storage subsystem in a shared enclosure, or such display devices may be peripheral display devices such as the display component shown in FIG. 2.

Communication subsystem 212 may be configured to communicatively couple the computing subsystem with one or more other computing devices. Communication subsystem 212 may include wired and/or wireless communication devices compatible with one or more different communication protocols. As non-limiting examples, the communication subsystem may be configured for communication via a wireless telephone network, or a wired or wireless local- or wide-area network. In some embodiments, the communication subsystem may allow the computing subsystem to send and/or receive data, messages, and/or the like to and/or from other devices via a network such as the internet.

### UTILITY

The methods of the present disclosure find use a variety of contexts, and in certain instances, enhance one or more aspects of a subject's cognition (e.g., one or more aspects of cognitive control) and/or physical abilities. Deficits in cognitive control, which dictate our ability to accomplish selected goals, can lead to significant impairments in cognition, social conduct, and affect regulation, and likely play an important role in a range of mental illnesses including ADHD, PTSD, Major Depressive Disorder, Traumatic Brain Injury, Obsessive Compulsive Disorder, and Substance Dependence Disorders. Accordingly, in addition to enhancing cognition, the methods of the present disclosure find use in treating and diagnosing subjects having cognitive disorders.

The embodied cognitive tasks of the methods of the present disclosure are single tasks that require both a cognitive response and a physical response from the subject. Cognitive and physical training approaches have been developed in recent years, with some groups considering the benefits of coupling cognitive tasks and physical tasks together. However, the cognitive and physical challenges in the existing approaches are not integrated. See, e.g., Goji Play^{™}, in which a person plays a video game while engaging in physical exercise (e.g., riding an exercise bike, etc.), but the game and exercise are independent/non-integrated. Without being bound by theory, it is believed that aspects of the methods of the present disclosure which involve embodied cognitive tasks that couple cognitive and physical challenges enhances cognition to a greater degree than approaches in which the cognitive and physical challenges are independent/non-integrated. Moreover, in certain aspects, the present methods prevent tradeoffs by tethering adaptation and/or rewards to performance on both the cognitive and physical fronts - an advantageous feature absent from previous training approaches.

Approaches for enhancing cognition include self-guided training sessions. However, self-guided training fails to provide the trainee with feedback and performance-based adaptation of difficulty levels for tracking progress and maximizing the effectiveness of the training. The methods of the present disclosure make up the deficiencies of self-guided approaches by providing the trainee with performance-based adaptation (and optionally, feedback), such that the methods of the present disclosure can be more effective at enhancing cognition as compared to self-guided approaches.

The following example is offered by way of illustration and not by way of limitation.

### EXPERIMENTAL

### EXAMPLE 1

Provided below is an example according to one embodiment of the present disclosure of a presentation of an embodied cognitive task, assessment of a subject's performance on the cognitive and physical components of the task, and adapting the difficulty level of the task based on the subject's performance on the cognitive and physical components. In this example, the embodied cognitive task is presented as a video game in which the subject's movements are detected using a Microsoft Kinect motion capture device.

A subject stands in front of an 85" plasma TV. The subject wears a heart monitor such as a Polar H1 heart rate sensor. The game unfolds with a story and challenge to motivate the subject. Next, the subject goes through a set of warmups to increase the heart rate and stretch the muscles. After this, the subject plays a thresholding game to assess his/her response time to perceptual challenges, with targets appearing at different distances from his/her center position. The subject needs to reach left/right, reach up/down, lunge left/right, and/or jump/duck depending on how far the targets appear. The thresholding data is used to guide the starting levels of the game.

Once the game begins, a 4 min counter starts to count down time for the first session. This particular module challenges selective attention and task switching for the cognitive components, and aerobic fitness for the physical component. The subject makes decisions about a cue that appears in the center of the screen. In this example, the cue is either a colored target (e.g., blue or green) or it will be a type of vegetable (e.g., radish or carrot). Seconds later, a set of targets appear around the subject (blue circle, green circle, radish, carrot). The subject needs to reach for the one that was cued to them as rapidly and accurately as possible. Then the subject returns to center position and the next cue appears.

There are three adaptive loops that change the game in response to the subject's performance. As he/she releases from the center position faster, the perceptual decision becomes more difficult, e.g., the colors morph together and the vegetables morph together. As he/she successfully makes it all the way and hits the target within a given period of time (established by the pre-thresholding procedure), the time in which he/she must do that becomes less. As the subject's heart rate remains lower than the level that was set before the game started (e.g., 120-140bpm: established using a pre-determined V0₂ Max to determine the subject's 70%-80% heart rate max), the distance from center that the target appears increases. Once the subject's heart rate goal is reached, the targets do not move farther, and if the heart rate goal is exceeded, the target's distance from center decreases. The subject plays the game for 4 minutes in this manner adaptively challenging themselves cognitively and physically on the embodied cognitive task presented as an integrated game platform. After the 4 minutes, the subject takes a break and, optionally, begins a next module with the same or different cognitive and physical challenges.

### EXAMPLE 2

Cognitive and physical training have shown positive effects on cognitive control abilities. We hypothesized that simultaneous cognitive and physical training would enhance the cognitive benefits. To test this, we developed a "Brain-Body Trainer" ("BBT") video game that combines these training approaches using video game mechanics, a Kinect for motion capture, and a heart rate monitor.

Healthy young adults (n=6) were recruited for the training study and for an expectancy-matched group of controls (n=10). Each participant completed a cognitive and physiological battery before and after training. BBT participants (5 females, 1 male) trained for 24 one hour-long sessions over eight weeks. Placebo participants (6 female, 4 male) trained for 15 hours over six weeks. The BBT was divided into three modules, each training a different aspect of cognitive control (attention, goal management, working memory), and each running for four minute intervals. In this example, the first module involved a visual search task for attention abilities. The second module involved a spatial span task for working memory. The third module involved a task switching paradigm for goal management abilities. Cognitive task difficulty adapted on a trial-by-trial basis, while physical task difficulty adapted in real-time - in this example, based on real-time heart rate measurements.

During the visual search module, participants were asked to reach to their left/right when indicated by the indicative cue within their personalized threshold period of time. They were also asked to reach up/down under the same circumstances; however, when reaching the up/down target, they were also then asked to run in place for a specified amount of time determined by their heart rate level and their VO2max prescribed fitness level. During the task switch module, participants were asked to reach left/right/up/down when indicated by the indicative cue within their personalized threshold period of time. Depending upon their heart rate level, the distance they were required to reach in any of these directions changed: if they were below their ideal heart rate, the required distance increased (and vice versa). For the working memory module, participants were instructed to which targets they needed to reach. Following a delay period, participants were asked to remember and reach to each of the targets from memory. Depending upon their heart rate level, the distance the participant needed to reach in any of these directions changed: if they were below their ideal heart rate, the distance increased (and vice versa).

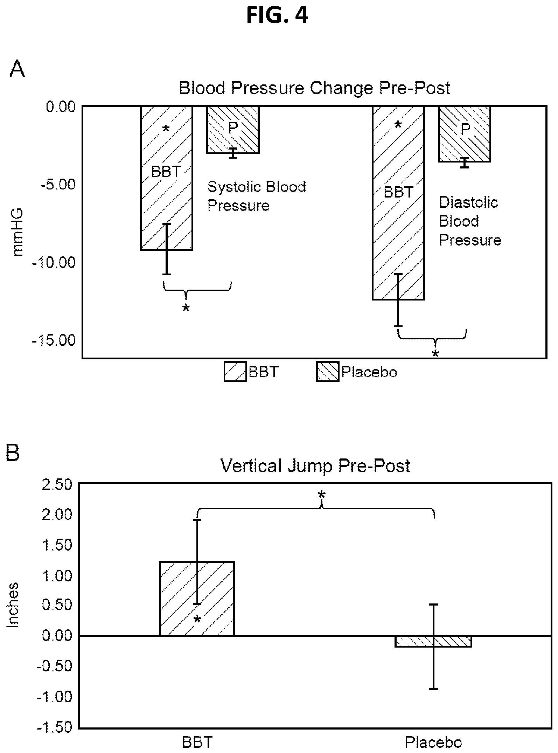

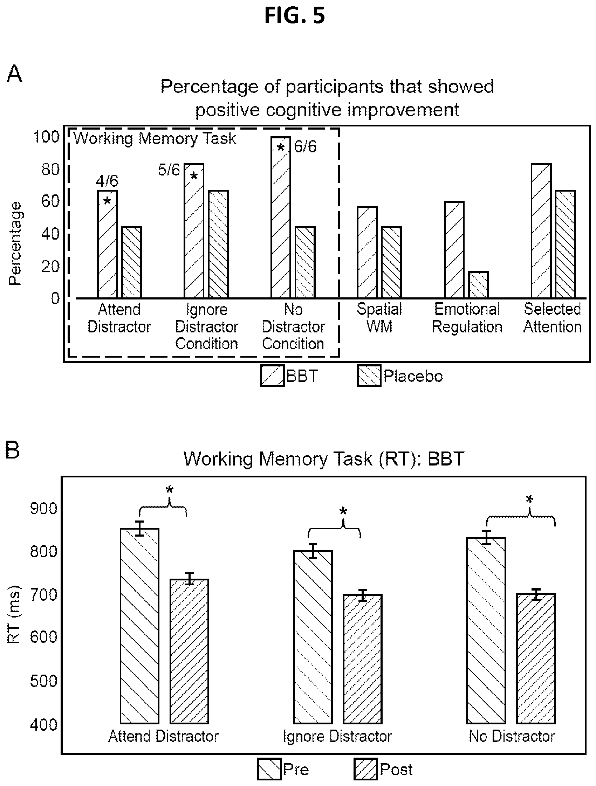

Physical and cognitive outcomes from the training study are shown in FIGs. 4 and 5, respectively. As shown in FIG. 4, panel A, a significant difference in systolic and diastolic blood pressure was observed between the BBT and placebo groups (* = p < .05). As shown in FIG. 4, panel B, a significant difference in vertical jump height was observed between the BBT and placebo groups (* = p < .05).

With respect to cognitive outcomes, a significant difference in the percentage of participants showing positive cognitive improvement, as determined by a working memory task, was observed between the BBT and placebo groups (FIG. 5, panel A) (* within box = significant paired t test). For the working memory task, BBT participants showed a significant reduction in reaction time post-training compared to pre-training (FIG. 5, panel B) (* = p < .05).

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of present invention is embodied by the appended claims.

## Claims

1. A method of enhancing cognition in a subject, the method being implemented using a computer system (200, 300) having a display component (208, 306), the method comprising:
the computer system (200, 300) presenting to a subject an embodied cognitive task via the display component (208, 306), the embodied cognitive task comprising a cognitive component and a physical component, wherein the cognitive component comprises identification of a virtual target stimulus presented to the subject via the display component (208, 306), and wherein the physical component comprises reaching for the virtual target stimulus, requiring body movement by the subject;
the computer system assessing the subject's performance on each of the cognitive component and the physical component of the embodied cognitive task, wherein
assessing the subject's performance on the cognitive component comprises the computer system (200, 300) detecting the subject's body movement; and
assessing the subject's performance on the physical component comprises the computer system (200, 300) monitoring the subject's heart rate using a heart rate monitor (310); and
the computer system (200, 300) adapting the difficulty level of the embodied cognitive task based on the assessment to effect a change in the subject's subsequent performance on each of the cognitive component and the physical component.

2. The method according to Claim 1, wherein the display component (208, 306) is selected from the group consisting of: a television, a monitor, a high-definition television (HDTV), a projection screen, and a head-up display (HUD).

3. The method according to Claim 1 or Claim 2, wherein the embodied cognitive task is a video game.

4. The method according to any one of Claims 1 to 3, wherein the cognitive component presented by the computer system (200, 300) targets a cognitive ability of the subject selected from the group consisting of: working memory, attention, task-switching, goal management, target search, target discrimination, and any combination thereof.

5. The method according to any one of claims 1 to 4, wherein:
the body movement of the subject detected by the computer system (200, 300) indicates a reaction time to the cognitive component of the embodied cognitive task, and/or
the body movement of the subject detected by the computer system (200, 300) indicates a response accuracy to the cognitive component of the embodied cognitive task, and/or
the body movement is detected by the computer system (200, 300) using motion sensors worn on the subject's body, and/or
the body movement is detected by the computer system using a motion capture device (210, 304), the motion capture device (210, 304) optionally comprising a depth camera.

6. The method according to any one of Claims 1 to 5, wherein the subject's performance on the cognitive component is further assessed by the computer system (200, 300) by detecting neural activity of the subject, optionally wherein the neural activity is detected using a neural activity detection method selected from the group consisting of: electroencephalography (EEG), near-infrared spectroscopy (NIRS), optical imaging, functional magnetic resonance imaging (fMRI), and any combination thereof.

7. The method according to any one of Claims 1 to 6, wherein the physical component presented by the computer system (200, 300) targets a physical ability of the subject selected from the group consisting of: the subject's aerobic energy-generating ability, balance, flexibility, coordination, and any combination thereof.

8. The method according to any one of Claims 1 to 7, wherein the subject's performance on the physical component is further assessed by the computer system (200, 300) by detecting body movement of the subject, optionally wherein the body movement is detected by the computer system using motion sensors worn on the subject's body, and/or a motion capture device, and/or a depth camera.

9. The method according to any one of Claims 1 to 8, wherein the subject's performance on the physical component is further assessed by the computer system (200, 300) by detecting the physiological measure of the subject, optionally wherein the physiological measure is selected from the group consisting of: heart rate, heart rate variability, VO₂ Max, electrodermal response (EDR), pupillary response, and any combination thereof.

10. The method according to any one of Claims 1 to 9, wherein the subject has a cognitive disorder selected from the group consisting of: attention deficit hyperactivity disorder (ADHD), traumatic brain injury (TBI), post-traumatic stress disorder (PTSD), obsessive compulsive disorder (OCD), substance dependence disorder (SDD), depression, dementia, Alzheimer's disease, Parkinson's disease, autism, and schizophrenia.

11. The method according to any one of Claims 1 to 9, wherein the subject is a healthy subject.

12. The method according to Claim 11, wherein the healthy subject is from an age group selected from the group consisting of: preschool, middle childhood, young teen, teenager, younger adult, middle-age adult, and older adult.

13. A non-transitory computer-readable medium comprising instructions stored thereon for causing a computer system (200, 300) to implement the method of any one of Claims 1 to 12.

14. A computer system (200, 300) comprising the non-transitory computer-readable medium of Claim 13.

## Patentansprüche

1. Verfahren zur Verbesserung der Kognition eines Individuums, wobei das Verfahren unter Verwendung eines Computersystems (200, 300) implementiert wird, das eine Anzeigekomponente (208, 306) aufweist, wobei das Verfahren Folgendes umfasst:
Präsentieren, durch das Computersystem (200, 300), einer eingebetteten kognitiven Aufgabe für das Individuum über die Anzeigekomponente (208, 306), wobei die eingebettete kognitive Aufgabe eine kognitive Komponente und eine körperliche Komponente umfasst, wobei die kognitive Komponente die Identifikation eines virtuellen Zielstimulus umfasst, die dem Individuum über die Anzeigekomponente (208, 306) präsentiert wird, und wobei die körperliche Komponente das Greifen nach dem virtuellen Zielstimulus umfasst, was eine Körperbewegung durch das Individuum erfordert;
Beurteilen, durch das Computersystem, der Leistung des Individuums bei jeder aus der kognitiven Komponente und der körperlichen Komponente der eingebetteten kognitiven Aufgabe, wobei
das Beurteilen der Leistung des Individuums hinsichtlich der kognitiven Komponente umfasst, dass das Computersystem (200, 300) die Körperbewegung des Individuums detektiert; und
das Beurteilen der Leistung des Individuums hinsichtlich der körperlichen Komponente umfasst, dass das Computersystem (200, 300) die Herzrate des Individuums unter Verwendung einer Herzratenüberwachungsvorrichtung (310) überwacht; und
Anpassen, durch das Computersystem (200, 300), des Schwierigkeitsgrads der eingebetteten kognitiven Aufgabe basierend auf der Beurteilung, um eine Änderung der nachfolgenden Leistung des Individuums hinsichtlich jeder aus der kognitiven Komponente und der körperlichen Komponente zu bewirken.

2. Verfahren nach Anspruch 1, wobei die Anzeigekomponente (208, 306) aus folgender Gruppe ausgewählt ist: einem Fernsehgerät, einem Bildschirm, einem hochauflösenden Fernsehgerät (HDTV), einem Projektionsschirm und einem Head-up-Display (HUD).

3. Verfahren nach Anspruch 1 oder Anspruch 2, wobei die eingebettete kognitive Aufgabe ein Videospiel ist.

4. Verfahren nach einem der Ansprüche 1 bis 3, wobei die kognitive Komponente, die vom Computersystem (200, 300) präsentiert wird, auf eine kognitive Fähigkeit des Individuums abzielt, die aus folgender Gruppe ausgewählt ist: Arbeitsgedächtnis, Aufmerksamkeit, Aufgabenwechsel, Zielmanagement, Zielsuche, Zielunterscheidung und einer beliebigen Kombination davon.

5. Verfahren nach einem der Ansprüche 1 bis 4, wobei:
die Körperbewegung des Individuums, die vom Computersystem (200, 300) detektiert wird, eine Reaktionszeit auf die kognitive Komponente der eingebetteten kognitiven Aufgabe angibt und/oder
die Körperbewegung des Individuums, die vom Computersystem (200, 300) detektiert wird, eine Reaktionsgenauigkeit auf die kognitive Komponente der eingebetteten kognitiven Aufgabe angibt und/oder
die Körperbewegung vom Computersystem (200, 300) unter Verwendung von Bewegungssensoren detektiert wird, die am Körper des Individuums getragen werden, und/oder
die Körperbewegung vom Computersystem unter Verwendung einer Bewegungserfassungsvorrichtung (210, 304) detektiert wird, wobei die Bewegungserfassungsvorrichtung (210, 304) gegebenenfalls eine Tiefenkamera umfasst.

6. Verfahren nach einem der Ansprüche 1 bis 5, wobei die Leistung des Individuums hinsichtlich der kognitiven Komponente weiters vom Computersystem (200, 300) beurteilt wird, indem die Nervenaktivität des Individuums detektiert wird, wobei die Nervenaktivität gegebenenfalls unter Verwendung eines Nervenaktivitätsdetektionsverfahrens detektiert wird, das aus folgender Gruppe ausgewählt ist: Elektroenzephalographie (EEG), Nahinfrarotspektroskopie (NIRS), optische Bildgebung, funktionelle Magnetresonanztomographie (fMRI) und einer beliebigen Kombination davon.

7. Verfahren nach einem der Ansprüche 1 bis 6, wobei die körperliche Komponente, die vom Computersystem (200, 300) präsentiert wird, auf eine körperliche Fähigkeit des Individuums abzielt, die aus folgender Gruppe ausgewählt ist: aerober Energiebereitstellungsfähigkeit des Individuums, Gleichgewicht, Flexibilität, Koordination und einer beliebigen Kombination davon.

8. Verfahren nach einem der Ansprüche 1 bis 7, wobei die Leistung des Individuums hinsichtlich der körperlichen Komponente weiters vom Computersystem (200, 300) beurteilt wird, indem es eine Körperbewegung des Individuums detektiert, wobei die Körperbewegung vom Computersystem gegebenenfalls unter Verwendung von Bewegungssensoren, die am Körper des Individuums getragen werden, und/oder einer Bewegungserfassungsvorrichtung und/oder einer Tiefenkamera erfasst wird,

9. Verfahren nach einem der Ansprüche 1 bis 8, wobei die Leistung des Individuums hinsichtlich der körperlichen Komponente weiters vom Computersystem (200, 300) beurteilt wird, indem es einen physiologischen Messwert des Individuums detektiert, wobei der physiologische Messwert gegebenenfalls aus folgender Gruppe ausgewählt ist: Herzrate, Herzratenvariabilität, VO₂ max, elektrodermaler Reaktion (DER), Pupillenreaktion und einer beliebigen Kombination davon.

10. Verfahren nach einem der Ansprüche 1 bis 9, wobei das Individuum eine kognitive Störung aufweist, die aus folgender Gruppe ausgewählt ist:
Aufmerksamkeitsdefizit-/Hyperaktivitätsstörung (ADHS), Schädel-Hirn-Traums (SHT), posttraumatischer Belastungsstörung (PTBS), Zwangsstörung (OCD), Substanzabhängigkeit (SA), Depression, Demenz, Alzheimer-Krankheit, Parkinson-Krankheit, Autismus und Schizophrenie.

11. Verfahren nach einem der Ansprüche 1 bis 9, wobei das Individuum ein gesundes Individuum ist.

12. Verfahren nach Anspruch 11, wobei das gesunde Individuum zu einer Altersgruppe gehört, die aus folgender Gruppe ausgewählt ist: Vorschule, mittlerer Kindheit, jungen Teenagern, Teenagern, jungen Erwachsenen, Erwachsenen mittleren Alters und älteren Erwachsenen.

13. Nichtflüchtiges, computerlesbares Medium, auf dem Anweisungen gespeichert sind, die bewirken, dass ein Computersystem (200, 300) ein Verfahren nach einem der Ansprüche 1 bis 12 ausführt.

14. Computersystem (200, 300), das ein nichtflüchtiges, computerlesbares Medium nach Anspruch 13 umfasst.

## Revendications

1. Procédé d'amélioration de la cognition chez un sujet, le procédé étant mis en œuvre à l'aide d'un système informatique (200, 300) présentant un composant d'affichage (208, 306), le procédé comprenant :
le système informatique (200, 300) présentant à un sujet une tâche cognitive incarnée via le composant d'affichage (208, 306), la tâche cognitive incarnée comprenant une composante cognitive et une composante physique, dans lequel la composante cognitive comprend l'identification d'un stimulus cible virtuel présenté au sujet via le composant d'affichage (208, 306), et dans lequel la composante physique comprend l'atteinte du stimulus cible virtuel, nécessitant un mouvement du corps par le sujet ;
le système informatique évaluant les performances du sujet sur chacun de la composante cognitive et de la composante physique de la tâche cognitive incarnée, dans lequel
l'évaluation de la performance du sujet sur la composante cognitive comprend le système informatique (200, 300) détectant le mouvement du corps du sujet ; et
l'évaluation de la performance du sujet sur la composante physique comprend le système informatique (200, 300) surveillant la fréquence cardiaque du sujet à l'aide d'un dispositif de surveillance de fréquence cardiaque (310) ; et
le système informatique (200, 300) adaptant le niveau de difficulté de la tâche cognitive incarnée sur la base de l'évaluation pour effectuer un changement dans la performance ultérieure du sujet sur chacun de la composante cognitive et de la composante physique.

2. Procédé selon la revendication 1, dans lequel le composant d'affichage (208, 306) est choisi dans le groupe constitué : d'une télévision, d'un moniteur, d'une télévision à haute définition (HDTV), d'un écran de projection et d'un affichage tête haute (HUD).

3. Procédé selon la revendication 1 ou la revendication 2, dans lequel la tâche cognitive incarnée est un jeu vidéo.

4. Procédé selon l'une quelconque des revendications 1 à 3, dans lequel la composante cognitive présentée par le système informatique (200, 300) cible une capacité cognitive du sujet choisie dans le groupe comprenant : mémoire de travail, attention, commutation de tâches, gestion d'objectifs, recherche de cible, discrimination de cible et toute combinaison de celles-ci.

5. Procédé selon l'une quelconque des revendications 1 à 4, dans lequel :
le mouvement de corps du sujet détecté par le système informatique (200, 300) indique un temps de réaction à la composante cognitive de la tâche cognitive incarnée, et/ou
le mouvement de corps du sujet détecté par le système informatique (200, 300) indique une précision de réponse à la composante cognitive de la tâche cognitive incarnée, et/ou
le mouvement de corps est détecté par le système informatique (200, 300) à l'aide de capteurs de mouvement portés sur le corps du sujet, et/ou
le mouvement de corps est détecté par le système informatique à l'aide d'un dispositif de capture de mouvement (210, 304), le dispositif de capture de mouvement (210, 304) comprenant facultativement une caméra de profondeur.

6. Procédé selon l'une quelconque des revendications 1 à 5, dans lequel la performance du sujet sur la composante cognitive est en outre évaluée par le système informatique (200, 300) en détectant l'activité neuronale du sujet, facultativement dans lequel l'activité neuronale est détectée en utilisant un procédé de détection d'activité neuronale choisi dans le groupe comprenant : électroencéphalographie (EEG), spectroscopie dans le proche infrarouge (NIRS), imagerie optique, imagerie par résonance magnétique fonctionnelle (IRMf), et toute combinaison de celles-ci.

7. Procédé selon l'une quelconque des revendications 1 à 6, dans lequel la composante physique présentée par le système informatique (200, 300) cible une capacité physique du sujet choisie dans le groupe comprenant : la capacité de génération d'énergie aérobie du sujet, l'équilibre, la flexibilité, la coordination et toute combinaison de ceux-ci.

8. Procédé selon l'une quelconque des revendications 1 à 7, dans lequel la performance du sujet sur la composante physique est en outre évaluée par le système informatique (200, 300) en détectant le mouvement de corps du sujet, facultativement dans lequel le mouvement de corps est détecté par le système informatique en utilisant des capteurs de mouvement portés sur le corps du sujet, et/ou un dispositif de capture de mouvement, et/ou une caméra de profondeur.

9. Procédé selon l'une quelconque des revendications 1 à 8, dans lequel la performance du sujet sur la composante physique est en outre évaluée par le système informatique (200, 300) en détectant la mesure physiologique du sujet, facultativement dans lequel la mesure physiologique est choisie dans le groupe comprenant : la fréquence cardiaque, la variabilité de la fréquence cardiaque, la VO₂ Max, la réponse électrodermique (EDR), la réponse pupillaire, et toute combinaison de celles-ci.

10. Procédé selon l'une quelconque des revendications 1 à 9, dans lequel le sujet présente un trouble cognitif choisi dans le groupe comprenant : un trouble d'hyperactivité avec déficit de l'attention (TDAH), une lésion cérébrale traumatique (TBI), un trouble de stress post-traumatique (TSPT), un trouble obsessionnel compulsif (TOC), un trouble de dépendance à une substance (TDS), une dépression, une démence, la maladie d'Alzheimer, la maladie de Parkinson, l'autisme et la schizophrénie.

11. Procédé selon l'une quelconque des revendications 1 à 9, dans lequel le sujet est un sujet sain.

12. Procédé selon la revendication 11, dans lequel le sujet sain appartient à un groupe d'âge choisi dans le groupe comprenant : le préscolaire, le milieu de l'enfance, le jeune adolescent, l'adolescent, le jeune adulte, l'adulte d'âge moyen et l'adulte plus âgé.

13. Support non transitoire lisible par ordinateur comprenant des instructions stockées sur celui-ci pour amener un système informatique (200, 300) à mettre en œuvre le procédé selon l'une quelconque des revendications 1 à 12.

14. Système informatique (200, 300) comprenant le support lisible par ordinateur non transitoire selon la revendication 13.
